(19) Europäisches Patentamt
European Patent Office
Office européen des brevets

(11) **EP 3 650 436 A1**

(12) **EUROPEAN PATENT APPLICATION**

(43) Date of publication:
**13.05.2020  Bulletin 2020/20**

(21) Application number: **18461623.3**

(22) Date of filing: **06.11.2018**

(51) Int Cl.:
**C07C 67/333** (2006.01)          **C07C 69/533** (2006.01)
**C07C 6/04** (2006.01)              **C07C 11/02** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Skotan S.A.**
**41-506 Chorzow (PL)**

(72) Inventors:
• **Wyrebek, Przemyslaw**
  **30-333 Kraków (PL)**

• **Sytniczuk, Adrian**
  **05-300 Minsk Mazowiecki (PL)**
• **Kosnik, Wioletta**
  **07-311 Wasewo (PL)**
• **Malecki, Pawel**
  **05-831 Parole (PL)**
• **Grela, Karol Leslaw**
  **01-248 Warsyawa (PL)**

(74) Representative: **Witek, Rafal**
**WTS Patent Attorneys**
**Witek, Sniezko & Partners**
**ul. Weigla 12**
**53-114 Wroclaw (PL)**

(54) **METHOD OF PREPARING A MIXTURE OF 9-DECENOIC ACID ETHYL AND 1-DECENE IN AN ETHENOLYSIS REACTION FROM A RAW MATERIAL OF TECHNICAL PURITY**

(57)     The subject of the invention is a method for obtaining a mixture of ethyl 9-decenoate and 1-decene by ethenolysis reaction from a raw material of technical purity containing fatty acid alkyl esters and ethyl oleate content of at least 70%, comprising the following steps: a) preparation of the substrate - alkyl ester of oleic acid, b) preparation of the catalyst solution, c) contacting the catalyst solution with the substrate in the reactor, d) filling the reactor with ethylene gas, e) mixing the reaction mixture, f) pouring down the reaction mixture from the reactor, g) fractional distillation of the reaction mixture, h) hydrolysis of 9-decenoic acid alkyl esters, characterized in that in step a) the substrate is contacted with magnesol, and in step b) the catalyst solution is prepared by dissolving in toluene a ruthenium complex containing at least one carbene ligand with a five-membered heterocyclic ring containing two tertiary nitrogen atoms in which one of the nitrogen atoms is substituted with a thiophenomethylene substituent and the other nitrogen atom is substituted with an aromatic substituent containing at least one branched alkyl substituent containing at least three carbon atoms in the alkyl chain and the reaction in step e) is carried out for 2 to 8 hours and at a temperature of 50 °C to 80 °C under ethylene pressure 10 bar to 20 bar and in step f) the reaction mixture is contacted with the scavenger solution.

EP 3 650 436 A1

**Description**

The present invention relates to a method for obtaining ethyl 9-decenoate and 1-decene from a mixture comprising a mixture of oleic acid esters by an ethenolysis reaction, catalyzed by an organometallic ruthenium complex. The invention finds application in processes in which a metathesis reaction of long-chain hydrocarbons with multiple bonds occurs in the environment of low molecular weight unsaturated hydrocarbons, wherein starting materials are technical mixtures containing ethyl oleate.

US2007270621A1 discloses a process for producing chemicals from renewable sources. The known method uses readily available, renewable resources containing fatty acids. According to the disclosed description, 1-octene, methyl 9-decenoate and butadiene are prepared from linoleic acid subjected to enzymatic isomerization in the first step, followed by metathesis with ethylene. Linoleic acid is isolated from vegetable oils such as soybean oil. The ethenolysis reaction is carried out in a glove box in an inert atmosphere. A commercially available first or second generation catalyst is used for the metathesis reaction.

In another document, US2014336399A1, a method for producing fuel from substrates of natural origin is described. The described method involves reacting the feedstock with a molar excess of low molecular weight olefins in the presence of a metathesis catalyst. The cross-metathesis reaction in the first step is carried out directly with the glyceride substrate in the presence of a ruthenium catalyst ([1,3-bis-(2,4,6-trimethylphenyl)-2-imidazolidinylidene]dichlororuthenium-(3-methyl-2-butenylidene)(tricyclohexylphosphine) and a low molecular weight olefin The metathesis reaction proceeds for 2 hours at 60 °C. The reaction mixture is then cooled, decolorized with an adsorbent, filtered and transesterified with NaOMe in methanol at 60 °C. According to the method described in the cited document, undesirable products are also generated, such as: methyl 9-dodecenoate, methyl 9-dodecanoate, unsaturated diesters. The known method is characterized by low reaction selectivity, low yield of the main product (22-46%) and a significant proportion of additional products (impurities).

WO02076920A1 discloses a double olefin replacement process comprising contacting an unsaturated fatty acid ester or an unsaturated fatty acid, such as methyl oleate or oleic acid, with a lower olefin (e.g. ethylene) in the presence of a metathesis catalyst to produce an olefinic product, such as e.g. methyl 9-decenoate, 9-decenoic acid or 1-decene. The metathesis catalyst contains ruthenium or osmium and a chelating ligand with a carbene moiety and a second donor moiety with an element from the 15th or 16th group of the periodic table. In addition, the catalyst may be supported on a solid support, such as a crosslinked polymer resin. The metathesis reaction is carried out at a molar ratio of ethene: fatty acid ester from 0.8:1 to 20:1. Oleic acid was used as the fatty acid. In contrast, the catalyst, in addition to the substituted carbene ligand, contained halides directly bound to the ruthenium atom and tricyclohexylphosphine (PCy$_3$). The metathesis reaction was carried out at 60 °C for 3 hours. The yields of 1-decene and methyl 9-decenoate did not exceed 50%. Only the addition of a stabilizing ligand resulted in an efficiency increase above 50%, but only after 23 hours. The process described in the cited document is characterized by too long reaction time to achieve satisfactory efficiency from the point of view of usability and industrial.
WO2011056881A2 discloses a catalyst comprising a combination of a cyclic alkylcarbene ligand and benzylidene and describes a method for obtaining linear alpha-olefins using it. The benzylidene group contains a electron-withdrawing substituent SO$_2$N-R$_2$ at the 3-position. In contrast, the carbene ligand is built on a pyrrolidine ring where the nitrogen atom is substituted with a phenyl ring containing alkyl substituents or only hydrogen atoms or a combination thereof, wherein the carbon atom at position 3 is substituted with alkyls. In a known method, a SO$_2$N-R$_2$-type catalyst is contacted with a mixture containing triacylglycerides, fatty acids, fatty acid alkyl esters .usually prepared from seeds with a high oil content. As a result of the ethenolysis reaction, a mixture of 1-decene and methyl 9-decenote is obtained. The method disclosed in the document primarily describes the process for obtaining olefins from natural mixtures, and methyl 9-decenoate is only an additional product. In addition, the catalyst used has a high selectivity at the expense of efficiency and a number of catalytic cycles (TON).

EP3294747A1 discloses catalysts containing metallic center (ruthenium) combined with a cyclic carbene ligand for use in broadly understood organic synthesis, using olefin cross-metathesis reactions, metathetic ring closure, metathetic ring closure of alkenes, in metathetic diastereoselective ring rearrangement reaction, olefin ring-opening polymerization and diene polymerization. The disclosed example relates to the ethenolysis reaction using chemically pure substrates, which is difficult to achieve under industrial practice conditions, and the purification process to the desired degree of purity can be economically unprofitable.
In the light of the prior art, there is a need to provide a method for obtaining 9-decenoic acid and 1-decene in one process from raw materials of technical purity, which method would be characterized by high selectivity of the reaction and low catalyst content to substrates ratio. From an industrial point of view, it is also desirable that the process proceeds with a high overall yield of final products. Another advantageous feature should be the ability to perform the preparation process without the need to use glove boxes (inert conditions) and techniques usually used in research laboratories that work with compounds particularly sensitive to oxygen and water.

Unexpectedly, such a method was obtained in the present invention.

The subject of the invention is a method for obtaining a mixture of ethyl 9-decenoate and 1-decene by the ethenolysis reaction from a raw material of technical purity and containing fatty acid alkyl esters and an ethyl oleate content of at least 70%, comprising the following steps:

> a) preparation of the substrate - alkyl ester of oleic acid,
> b) preparation of the catalyst solution,
> c) contacting the catalyst solution with the substrate in the reactor,
> d) filling the reactor with ethylene gas,
> e) mixing the reaction mixture,
> f) pouring down the reaction mixture from the reactor,
> g) fractional distillation of the reaction mixture,
> h) hydrolysis of 9-decenoic acid alkyl esters,

characterized in that in step a) the substrate is contacted with magnesol, and in step b) the catalyst solution is prepared by dissolving in toluene a ruthenium complex containing at least one carbene ligand with a five-membered heterocyclic ring containing two tertiary nitrogen atoms in which one of the nitrogen atoms is substituted with a thiophenomethylene substituent and the other nitrogen atom is substituted with an aromatic substituent containing at least one branched alkyl substituent containing at least three carbon atoms in the alkyl chain and the reaction in step e) is carried out for 2 to 8 hours and at a temperature of 50 °C to 80 °C under ethylene pressure 10 bar to 20 bar and in step f) the reaction mixture is contacted with the scavenger solution.

Preferably, ruthenium complex is selected from the group consisting of: bis[1-(2,6-diisopropylphenyl)-3-(thiophen-2-ylmethyl)imidazolidin-2-yl](3-phenyl-1H-inden-1-ylidene) ruthenium (II) chloride or [1-(2,6-diisopropylphenyl)-3-(thiophen-2-ylmethyl)imidazolidin-2-yl](2-isopropoxybenzylidene) ruthenium (II) chloride.

Preferably, the branched alkyl substituent is an isopropyl substituent.

Preferably, in step b) a homogeneous catalyst solution is prepared in a solvent selected from the group comprising toluene or dichloromethane. Depending on the scale of the ethenolysis reaction, the catalyst is dissolved in dichloromethane (small scale, during the choice of catalysts) or toluene (large scale, when scaling the process). The catalyst is dissolved in the minimum amount of solvent necessary to obtain a homogeneous solution. The solvent is used only to evenly distribute the catalyst in the reaction mixture.

Preferably, the reaction is carried out at a temperature of up to 50 °C in the presence of a monocarbene catalyst.

Preferably, the reaction is carried out at a temperature of 80 °C in the presence of a biscarbene catalyst.

Preferably, the reaction is carried out for up to 2 hours in the presence of a monocarbene catalyst.

Preferably, the reaction is carried out for up to 8 hours in the presence of a biscarbene catalyst.

Preferably, the reaction is carried out at ethylene pressure of 20 bar.

Preferably, the scavenger is 1,4-bis(3-isocyanopropyl)piperazine.

The analysis of technical samples of ethyl oleate was carried out. GC and GC/MS analysis showed that the purity of an ester (the sum of two geometrical isomers) is 71-72% (GC, peaks with $R_t$ = 33.38 min and $R_t$ = 33.44 min). The measurements were carried out on Perkin Elmer gas chromatographs: (GC) Clarus 580 and (GC/MS) Clarus 680 with a Clarus SQ 8C mass detector, using the InertCap SMS/Sil column and the same furnace temperature build-up. Based on the GC/MS measurement, it was found that the composition of the sample representative for series is a mixture of 6 compounds, two of which are the desired isomers of ethyl oleate. The mass spectrum for the peak with $R_t$ = 27.28 min indicates a compound with m/z 284 corresponding to the $C_{18}H_{36}O_2$ molecular formula (putative palmitic acid ethyl ester), the next peak with $R_t$ = 31.77 min indicates the derivative with m/z 308 probably corresponding to the linoleic ester derivative $C_{20}H_{36}O_2$. The next two peaks, $R_t$ = 32.07 min and $R_t$ = 32.12 min correspond to the isomers of oleic acid ethyl ester $C_{20}H_{38}O_2$ (m/z 310). In contrast, a peak with $R_t$ = 32.47 min corresponding to m/z 312 indicates a compound of formula $C_{20}H_{40}O_2$ (putative stearic acid ethyl ester). The mass spectrum of the last peak with $R_t$ = 36.03 min indicates the compound with m/z 338 and the molecular formula $C_{22}H_{42}O_2$ (hypothetical eicosenoic acid ethyl ester).

In the initial phase of the experiments that led to the invention, in order to select the group of the best catalysts for ethyl oleate ethenololysis reaction, the following initial reaction conditions were established: temperature = 50 °C, reaction time = 3 h, ethylene pressure = 10 bar. Next, it was decided to develop a simple and standardized method of conducting experiments, starting from the preparation of the substrate (oleic acid ethyl ester), conducting the reaction in an autoclave, and ending with the sampling for analysis by gas chromatography. The reaction scale was 15 mmol, which corresponds to about 5.4 cm$^3$ of an ester. This volume (together with the internal standard of approx. 6.2 cm$^3$) allowed for an effective process to be carried out in the apparatus intended for it (dimensions of the glass insert for the autoclave and the mixing element). The scale of the experiment was matched to the hardware requirements. Each time, before the ethenolysis reaction, the substrate (ethyl oleate) was filtered on a thin chromatographic column (column diameter of about 1-2 cm) through a thin layer of aluminum oxide (2 cm, neutral, activity I) to remove possible trace impurities. The procedure for sampling for analysis was also unified to maintain similar concentrations of substrate and reaction products (Example 1).

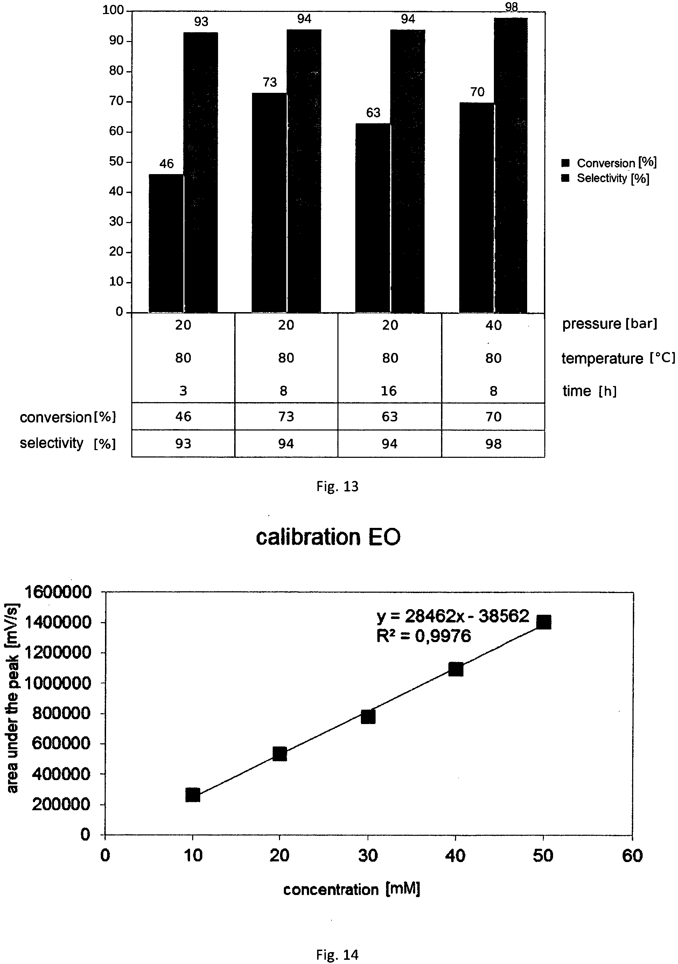

The efficiency of the catalysts was determined by means of gas chromatography. A standard curve was made for the EO in the concentration range of 5-50 mM. Samples of the reaction mixtures were diluted to 50 mM concentration and the substrate conversion was determined. For MOR, due to the overlap of substrate signals (mainly **1** and **7**), the calculation method was simplified (the calibration curve was abandoned and the internal standard addition method was used). A standard curve for ethyl oleate (EO) is shown in Fig. 14.

The by-products content generated in the EO homometathesis reaction was determined by taking their heats of combustion equal to the EO heat of combustion. Fractional distillation was carried out for selected reactions and the desired products were isolated.

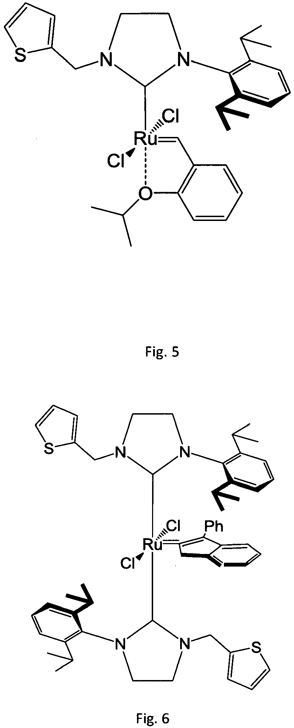

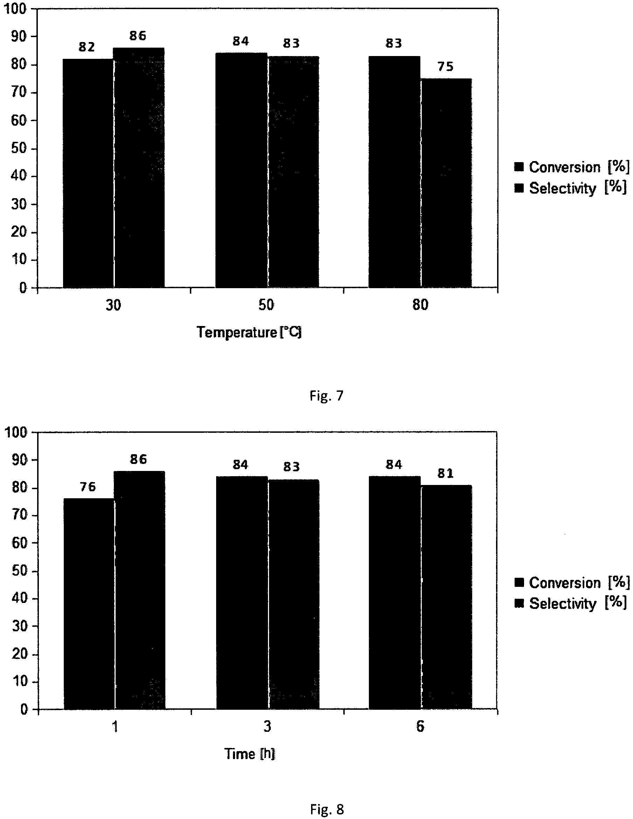

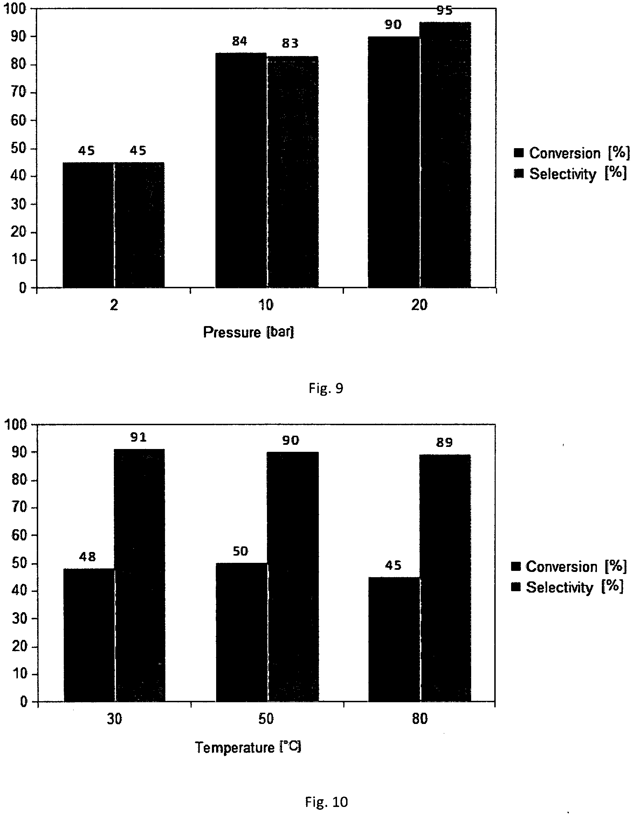

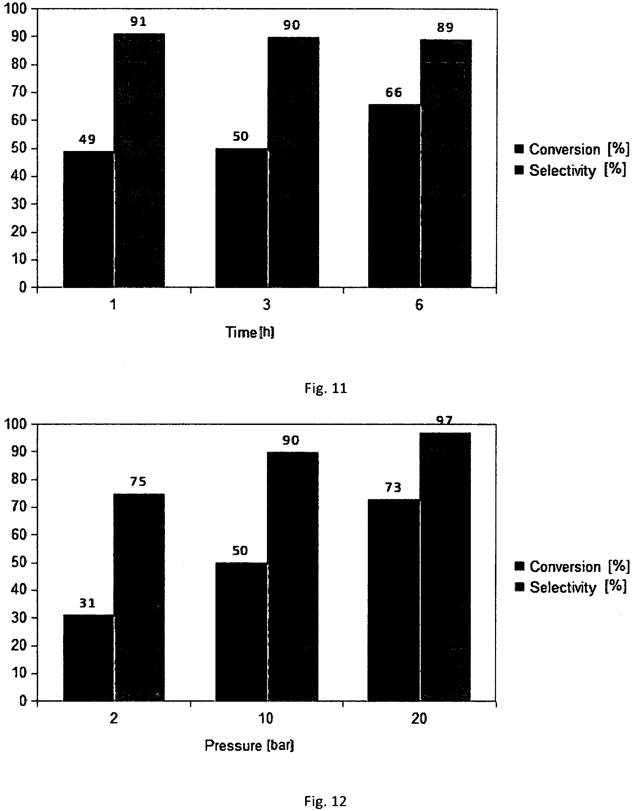

Embodiments of the invention are illustrated in the drawing, wherein the following figures show: Fig. 1: reaction scheme of ethyl oleate ethenolysis, Fig. 2: reaction scheme of MOR ethenolysis, Fig. 3: catalysts tested during the selection of the catalysts according to Example 1, Fig. 4: reference catalyst, Fig. 5: monocarbene catalyst [Ru]-63, Fig. 6: biscarbene catalyst, Fig. 7: optimization of the EO ethenolysis reaction temperature using a reference catalyst (reaction conditions: ([Ru]-18) (15 mmol, 200 ppm [Ru], 10 bar, 3h), Fig. 8: optimization of the EO ethenolysis reaction time using a reference catalyst (reaction conditions: ([Ru]-18) (15 mmol, 200 ppm [Ru], 10 bar, 50 °C), Fig. 9: optimization of ethylene pressure in the EO ethenolysis reaction using a reference catalyst ([Ru] -18) (15 mmol, 200 ppm [Ru], 3 h, 50 °C), Fig. 10: optimization of the EO ethenolysis reaction temperature using a monocarbene catalyst (15 mmol, 200 ppm [Ru], 10 bar, 3 h), Fig. 11: optimization of the EO ethenolysis reaction time using a monocarbene catalyst ([Ru]-63) (15 mmol, 200 ppm [Ru], 10 bar, 50 °C), Fig. 12: optimization of ethylene pressure in the EO ethenolysis reaction using a monocarbene catalyst ([Ru]-63) (15 mmol, 200 ppm [Ru], 3 h, 50 °C), Fig. 13: optimization of the EO ethenolysis reaction using a biscarbene catalyst (15 mmol, 200 ppm [Ru], 80 °C), Fig. 14: calibration curve for EO, Fig. 15: distillation chromatogram of ethyl oleate (1-decene fraction), Fig. 16: distillation chromatogram of ethyl oleate (mixed fraction of 1-decene and 9-decenoic acid ethyl ester), Fig. 17: distillation chromatogram of ethyl oleate (fraction of 9-decenoic acid ethyl ester), Fig. 18: MOR distillation chromatogram (first fraction of mainly 1-heptene), Fig. 19: MOR distillation chromatogram (second and third 1-decene fraction), Fig. 20: MOR distillation chromatogram (fourth mixed fraction of 1-decene and 1-decenoic acid methyl ester), Fig. 21: MOR distillation chromatogram (fifth, sixth and seventh fraction of 1-decenoic acid methyl ester).

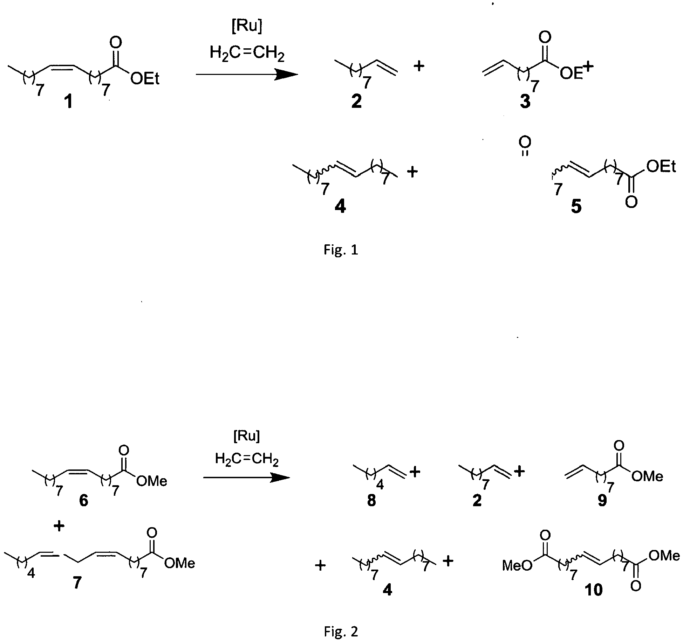

**Example 1. Ethenolysis using a reference catalyst (Fig. 1a), for comparison purposes.**

The oleic acid derivative (15 mmol) after filtration through a short column (bed height about 2 cm) with $Al_2O_3$ (neutral, activity I, 70-230 mesh, Merck) was placed in a glass autoclave insert. Then, 0.8 $cm^3$ of tetradecane was added as an internal standard. A catalyst solution (100 μl, 500 ppm, in DCM) was prepared in a separate vial and then added in one portion to the ethyl oleate insert. The autoclave was closed and connected to the ethylene line (purity 3.5, Linde), and then flushed three times with ethylene (by admitting and releasing ethylene from the system). The reaction was carried out for 3 hours at 50 °C under a pressure of 10 bar (with an open autoclave-gas connection). The ethylene was then released from the autoclave and a sample of the reaction mixture (1 $cm^3$) was collected, which was neutralized with a 2M solution of ethyl vinyl ether in DCM (4 $cm^3$). A sample for gas chromatography analysis was prepared by taking a sample of the obtained solution (0.2 $cm^3$) and filling up with DCM (0.8 cm3) to a volume of 1 $cm^3$.

**Example 2. Results of the catalyst selection for the ethyl oleate ethenolysis reaction (500 ppm [Ru], 10 bar, 3 h, 50 °C) carried out according to Example 1.**

| Catalyst | Conversion [%][a,b] | Yield **2** [%] [a,c] | Yield **3** [%] [a,c] | Selectivity [%][,a,b] |
|---|---|---|---|---|
| Ru-1 | 57 | 25 | 32 | 93 |
| Ru-2 | 67 | 44 | 51 | 93 |
| Ru-3 | 52 | 43 | 48 | 98 |
| Ru-4 | 65 | 31 | 38 | 90 |
| Ru-5 | 71 | 20 | 25 | 43 |
| Ru-6 | 77 | 41 | 55 | 76 |
| Ru-7 | 56 | 1 | 2 | 3 |
| Ru-8 | 38 | 17 | 22 | 40 |

(continued)

| Catalyst | Conversion [%][a,b] | Yield 2 [%] [a,c] | Yield 3 [%] [a,c] | Selectivity [%][,a,b] |
|---|---|---|---|---|
| Ru-9 | 79 | 47 | 58 | 70 |
| Ru-10 | 59 | 31 | 43 | 73 |
| Ru-11 | 53 | 9 | 11 | 17 |
| Ru-12 | 56 | 8 | 9 | 15 |
| Ru-13 | 80 | 51 | 57 | 72 |
| Ru-14 | 74 | 35 | 45 | 78 |
| Ru-15 | 77 | 41 | 47 | 66 |
| Ru-16 | 84 | 43 | 52 | 74 |
| Ru-17 | 70 | 46 | 60 | 89 |
| Ru-18 | 84 | 62 | 74 | 83 |
| Ru-19 | 42 | 39 | 42 | 99 |
| Ru-20 | 72 | 47 | 56 | 88 |
| Ru-21 | 57 | 12 | 14 | 26 |
| Ru-22 | 59 | 10 | 12 | 21 |
| Ru-23 | 81 | 10 | 19 | 43 |
| Ru-24 | 82 | 36 | 45 | 69 |
| Ru-25 | 74 | 29 | 35 | 54 |
| Ru-26 | 17 | 2 | 2 | 22 |
| Ru-27 | 39 | 13 | 16 | 38 |
| Ru-28 | 54 | 2 | 2 | 4 |
| Ru-29 | 40 | 6 | 7 | 23 |
| Ru-30 | 81 | 14 | 24 | 49 |
| Ru-31 | 60 | 14 | 15 | 28 |
| Ru-32 | 48 | 5 | 6 | 13 |
| Ru-33 | 58 | 17 | 20 | 32 |
| Ru-34 | 64 | 16 | 19 | 33 |
| Ru-35 | 38 | 5 | 5 | 17 |
| Ru-36 | 70 | 23 | 27 | 45 |
| Ru-37 | 54 | 4 | 5 | 8 |
| Ru-38 | 23 | 18 | 21 | 80 |
| Ru-39 | 57 | 5 | 6 | 12 |
| Ru-40 | 62 | 16 | 18 | 30 |
| Ru-41 | 64 | 15 | 18 | 31 |
| Ru-42 | 66 | 19 | 24 | 38 |
| Ru-43 | 59 | 9 | 11 | 22 |
| Ru-44 | 59 | 14 | 18 | 29 |
| Ru-45 | 63 | 6 | 7 | 14 |
| Ru-46 | 59 | 6 | 7 | 13 |

(continued)

| Catalyst | Conversion [%][a,b] | Yield 2 [%] [a,c] | Yield 3 [%] [a,c] | Selectivity [%][.a,b] |
|---|---|---|---|---|
| Ru-47 | 55 | 4 | 5 | 10 |
| Ru-48 | 55 | 2 | 3 | 5 |
| Ru-49 | 55 | 6 | 8 | 17 |
| Ru-50 | 54 | 8 | 6 | 15 |
| Ru-51 | 62 | 7 | 9 | 19 |
| Ru-52 | 55 | 10 | 13 | 21 |
| Ru-53 | 53 | 8 | 3 | 14 |
| Ru-54 | 72 | 30 | 37 | 55 |
| Ru-55 | 82 | 65 | 74 | 72 |
| Ru-56 | 81 | 62 | 71 | 80 |
| Ru-57 | 70 | 42 | 50 | 64 |
| Ru-58 | 68 | 36 | 43 | 75 |
| Ru-59 | 61 | 37 | 43 | 71 |
| Ru-60 | 61 | 31 | 38 | 67 |
| Ru-61 | 77 | 56 | 72 | 89 |
| Ru-62 | 39 | 23 | 28 | 78 |
| Ru-63 | 70 | 60 | 69 | 90 |
| Ru-64 | 42 | 39 | 34 | 99 |
| Ru-65 | 17 | 11 | 14 | 91 |

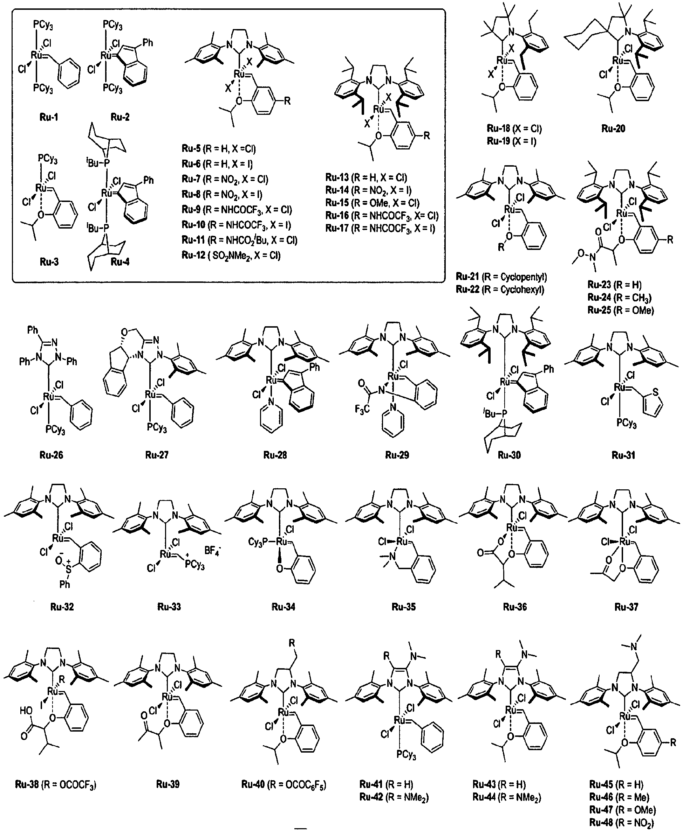

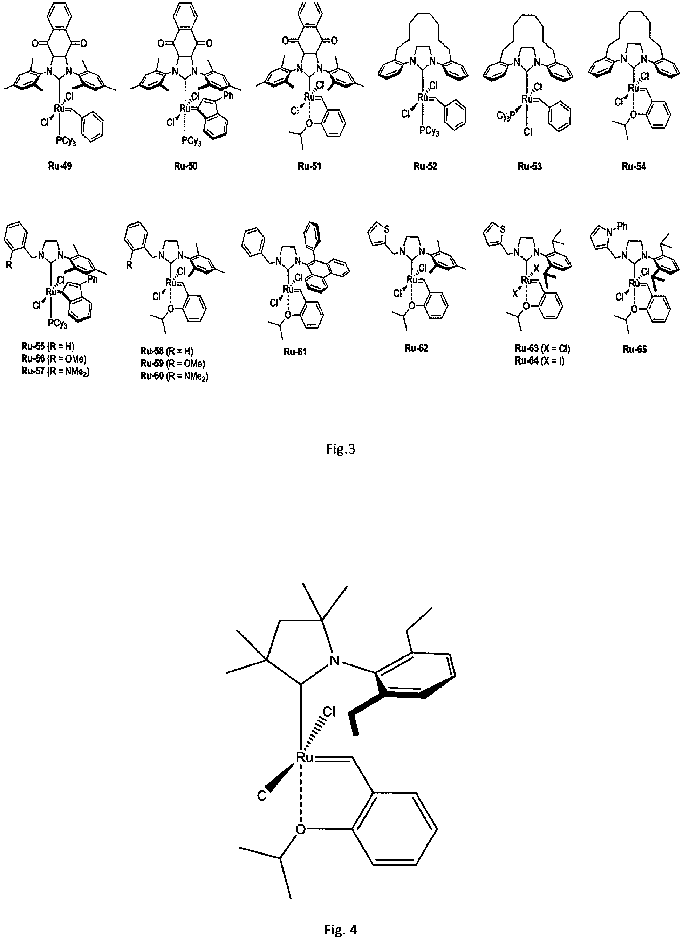

Numbering of the compounds of Fig. 3, where:
[a] Calculated based on GC measurements, by the addition of the standard (conversion, yield) or by the calibration curve method (selectivity).
[b] Conversion = 100 - 100 $\times$ (final number of moles of 1/initial number of moles of 1).
[c] Yield = 100 $\times$ number of moles of 2 or 3/initial number of moles of 1.
[d] Selectivity = 100 $\times$ (number of moles of 2 + number of moles of 3/[(number of moles of 2 + number of moles of 3) + 2 $\times$ (number of moles of 4 + number of moles of 5)

**Example 3. Preparation of methyl oleate (MOR).**

The MOR was mixed with silica gel (2% by weight) at the temperature of 110 °C and pressure of 1 mbar in a 60 liter reactor for 1-2 h. The reactor was then cooled, the MOR was decanted and filtered through silica plug using sintered glass funnel. The MOR was returned to the reactor and magnesol (5 wt%) was added thereto. The mixture was stirred for 12 h at 110 °C under a vacuum of 1 mbar. The next day, MOR was filtered through thin layer of magnesol using sintered glass funnel. The MOR thus prepared was mixed with activated alumina (5 wt%) overnight at 50 °C under high vacuum (0.05 mbar). The MOR was filtered through a layer of aluminum oxide to give a substrate for ethenolysis (a light yellow, with less intense odor).

**Example 4. Preparation of ethyl oleate (EO).**

Ethyl oleate (EO) was mixed with silica gel (2.5 wt%) at room temperature under argon for 1h, after which it was filtered through a thin layer of silica gel using sintered glass funnel. Then, OE was placed in a 60 dm$^3$ reactor and stirred together with magnesol (5 wt%) at 110 °C under high vacuum for 5 h. After cooling, magnesol was removed by filtration through thin layer of magnesol using sintered glass funnel. The EO was then stirred with activated alumina (2.5 wt%) for 2 h at room temperature under high vacuum, after which time activated carbon (2.5 wt%) was added to the flask. The mixture thus obtained was stirred under high vacuum at room temperature, overnight. The next day, EO was filtered through thin layer of magnesol using sintered glass funnel.

**Example 5. Ethenolysis procedure A.**

870 g of substrate (1000 cm$^3$) was weighed into flame-dried flask (volume: 2 dm$^3$) and degassed in vacuum at 50 °C for 0.5 h under stirring.

The catalyst and 2,6-dichloro-1,4-benzoquinone were weighed into 25 cm$^3$ flasks under vacuum and dissolved in 14 cm$^3$ of dry toluene under argon. The substrate was cooled to 0 °C and a solution of the catalyst (10 cm$^3$) and 2,6-dichloro-1,4-benzoquinone (5 cm$^3$) was added. The solution thus prepared was sucked into the reactor, inside of which a reduced pressure (1-5 mbar) was previously generated. During the introduction of the solution into the reactor, the pressure in the flask was compensated by the flow of argon to avoid sucking in air. Stirring was set at 600 rpm and ethylene was allowed in and out, twice, without exceeding the ethylene pressure for a given reaction. The reactions were carried out for 2 h at constant pressure, temperature and mixing values. After each full hour, sample was collected and analyzed by gas chromatography.

After 2 h, ethylene was removed and the reactor was flushed twice with argon, along with the line. The solution was poured into a vessel containing 4.4 equivalents, relative to the catalyst, of 1,4-bis(3-isocyanopropyl)piperazine.

Before the next ethenolysis reaction, the pressure reactor was washed with one portion of dichloromethane (800 cm$^3$), two portions of acetone (700 cm$^3$ each) and one portion of toluene (700 cm$^3$), stirring each time for 5 min (800 rpm). The reactor was dried under reduced pressure (1-5 mbar) at a jacket temperature of 65 °C.

**Example 6. Ethenolysis procedure B.**

870 g of substrate (1000 cm$^3$) was weighed into flame dried flask (2 dm$^3$) and degassed under vacuum at 50 °C for 0.5 h under stirring.

The catalyst was weighed into a 25 cm$^3$ flask in which the atmosphere was exchanged with argon and then dissolved in 14 cm$^3$ of dry toluene. The reactor, together with the gas supply line, were connected to the vacuum pump, draining the maximum amount of argon. The substrate was cooled below 0 °C and a catalyst solution (10 cm$^3$) was added. The solution was sucked into the reactor (jacket temperature equal to the reaction temperature), stirring was set at 600 rpm and ethylene was added to a given pressure. Reactions were carried out for 2 h, collecting the sample after each full hour.

After 2 h, ethylene was removed and reactor was flushed twice with argon, along with the line. The solution was poured into a vessel containing 4.4 equivalent, relative to the catalyst, of 1,4-bis(3-isocyanopropyl)piperazine.

Before the next ethenolysis reaction, the pressure reactor was washed with one portion of dichloromethane (800 cm$^3$), two portions of acetone (700 cm$^3$ each) and one portion of toluene (700 cm$^3$), stirring each time for 5 min (800 rpm). The reactor was dried under reduced pressure (1-5 mbar) at a jacket temperature of 65 °C.

**Example 7. Procedure for the reaction of a biscarbene complex (200 ppm [Ru], 8 h, 80 °C, 20 bar).**

Ethyl oleate (4.66 g, 15 mmol) after filtration through a short column (bed height about 2 cm) with Al$_2$O$_3$ (neutral, activity I) was placed in a glass autoclave insert. Then, 0.8 ml of tetradecane was added as internal standard. A catalyst solution (100 $\mu$l, 200 ppm, in DCM) was prepared in a separate vial and then added in one portion to the EO insert. The autoclave was closed and connected to the ethylene line (purity 3.0, Linde), and then flushed three times with ethylene (by admitting and releasing ethylene from the system). The reaction was carried out for 8 hours at 80 °C under a pressure of 20 bar (open gas cylinder-autoclave connection). Ethylene was then released from the autoclave and a sample of the reaction mixture (1 cm$^3$) was taken, which was neutralized with a 2M solution of ethyl vinyl ether in DCM (4 cm$^3$). The sample for gas chromatography analysis was prepared by taking a sample of the obtained solution (0.2 cm$^3$) and filling up with DCM (0.8 cm$^3$) to a volume of 1 cm$^3$.

The above procedure can be considered as general for all four reactions, taking into account appropriate changes in the pressure and duration of the reaction (it can be carried out according to example 8). The scale, catalyst loading, temperature, catalyst preparation and sample preparation for the analysis do not change.

Reaction according to the above example was carried out on a small scale and the post-reaction mixture was not distilled but only analyzed by GC.

**Example 8. Determination of substrate conversion by gas chromatography; sample concentration - 50 mM.**

The conversion rate was calculated from the following formula:

$$Conversion= \frac{GC\ PP\ [mV/s]}{WW(PP) \quad MOR(100PP)}{(62095) \quad (1386684)}$$

where: MOR(100PP) - GC area for 100% substrate, WW(PP) - GC area for internal standard, MOR(xPP) - GC area for starting materials in the post-reaction mixture.
MOR(100PP)/WW(PP): 22,33

$$Conversion[\%] = 100 - ([MOR(xPP)/WW(PP)]*100/22,33)$$

**Example 9. Effect of amount and type of catalyst on the efficiency of EO ethenolysis.**

Table 1. Effect of the reference catalyst amount ([Ru]-18, Fig. 3) on the efficiency of OE ethenolysis.[a]

| [Ru]-18 [ppm] | Conv. [%] | 5 [%] | 4 [%] | 2 [g] | 2 + 3 [g] | 3 [g] |
|---|---|---|---|---|---|---|
| 100 | 89 | 2,5 | 3,8 | 158,2 (97)[b] | 5,9 (82;12) | 246,1 (96) |
| 50 | 75 | 1,8 | 2,7 | 136,5 (98) | 3,3 (69;23) | 211,9 (97) |
| 25 | 54 | 1 | 1,4 | nd[c] | nd | nd |
| a - reactions were carried out according to the procedure "A", T = 40°C, p = 20 bar, t = 4h; b - GC purity (in the case of main fractions) or % content of the components in the mixed fractions are given in brackets; c - not distilled | | | | | | |

Table 2. Effect of the amount of monocarbene catalyst on the efficiency of EO ethenolysis.[a]

| [Ru]-63 [ppm] | Conv. [%] | 5 [%] | 4 [%] | 2 [g] | 2 + 3 [g] | 3 [g] |
|---|---|---|---|---|---|---|
| 100 | 43 | 0,6 | 1 | 68,3 (98) | 1,6 (59;33) | 107,1 (97) |
| 150 | 51 | 0,9 | 1,2 | Nd | nd | Nd |
| a - reactions were carried out according to the procedure "A", p = 20 bar, T = 50°C, t = 2h. | | | | | | |

**Example 10. Effect of the amount and type of monocarbene catalyst on the efficiency of MOR ethenolysis.**

Table 3. Effect of the amount of monocarbene catalyst on the efficiency of MOR ethenolysis.[a]

| [Ru]-63 [ppm] | Conv. [%] | 5 [%] | 4 [%] | Alkenes[b] [g] | 2[c] [g] | 2 + 9 [g] | 9 [g] | 9 + Z[d] |
|---|---|---|---|---|---|---|---|---|
| 150 | 77,9 (92,5) | 0,8 | 2,2 | 37,8 (16;60) | 76,9 (94) | 1,8 (57,23) | 184,1 (89) | 17,9 (65) |
| 100 | 69,3 (74,1) | 0,7 | 1,5 | 91,4 (23;58) | | 9,4 (63,25) | 147,4 (87) | 15,3 (81) |
| 50 | 52,1 (60,1) | 0,4 | 0,9 | 7,03 (70;6) | 59,2 (98) | 3,2 (54,31) | 119,5 (92) | 15,7 (52) |
| a - reactions were carried out according to the procedure "B", p = 20 bar, T = 50°C, t = 2h; b - mixture consisting of 2 and 8 in the % ratio given in brackets; c - one value in brackets indicates the GC purity of particular compound; d - higher boiling by-products, including 10. | | | | | | | | |

Table 4. MOR ethenolysis catalyzed by a reference complex ([Ru] -18).[a]

| Conv. [%] | 5 [%] | 4 [%] | Alkenes[b] [g] | 2[c] [g] | 2 + 9 [g] | 9 [g] | 9 + Z[d] |
|---|---|---|---|---|---|---|---|

(continued)

| Conv. [%] | 5 [%] | 4 [%] | Alkenes[b] [g] | 2[c] [g] | 2 + 9[g] | 9 [g] | 9 + Z[d] |
|---|---|---|---|---|---|---|---|
| 74,1(80,9) | 0,9 | 2,1 | 9,8 (68;1) | 83,3 (94) | 7,7 (57,30) | 168 (89) | 9(77) |

*a - reactions were carried out according to the procedure "B", 25 ppm, p = 20 bar, T = 50°C, t = 2 h; b - mixture consisting of 2 and 8 in the % ratio given in brackets; c - one value in brackets indicates the GC purity of particular compound; d - higher boiling by-products, including 5.*

## Example 11. Distillation of a post-reaction mixture after the ethyl oleate ethenolysis reaction.

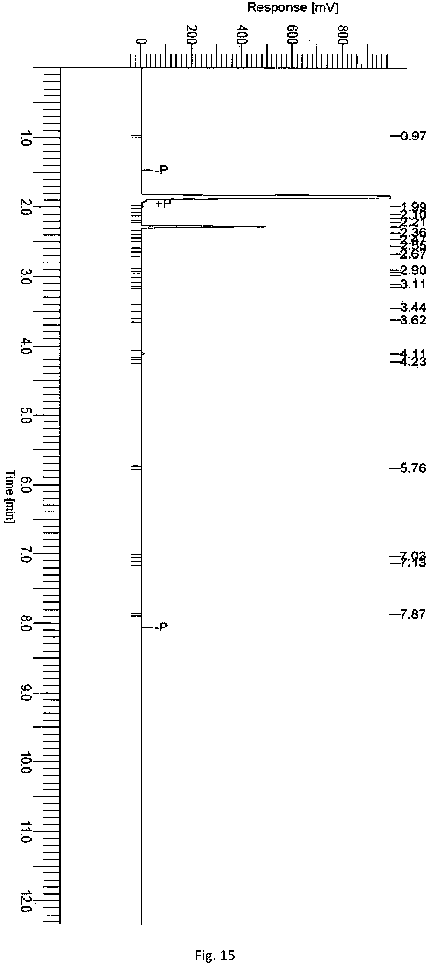

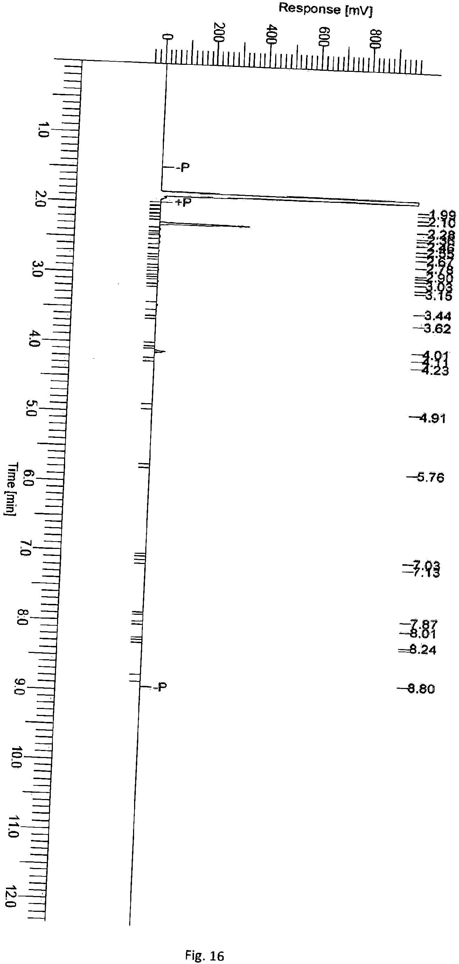

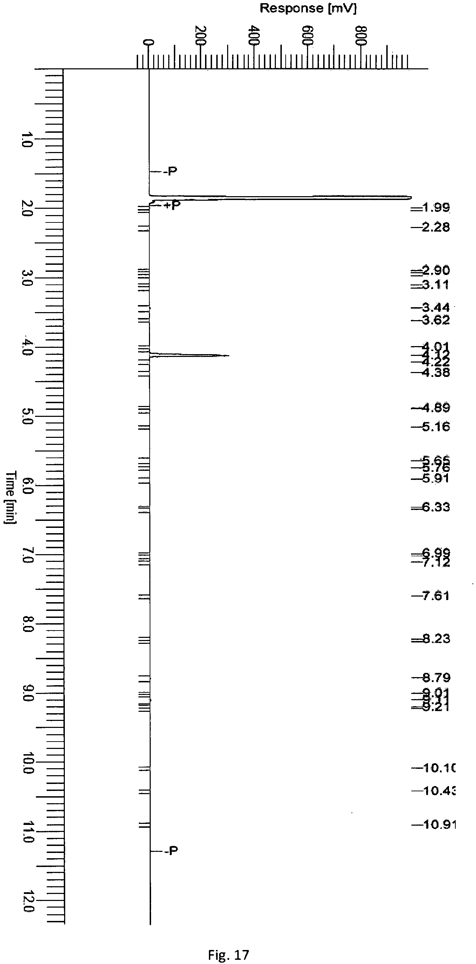

Distillation was carried out in a 1 $dm^3$ flask with 600 g of post-reaction mixture on a 44.5 cm distillation column. The entire distillation was carried out at a pressure of 1-2 mbar. All fractions were analyzed by GC using the following method: initial temperature 145 °C, time 0.1 min, heating 9 °C/min to 180 °C, time 0.1 min, heating 20 °C/min to 305 °C, time 2 minutes. Three fractions were collected: pure decene (purity > 95%) (Fig. 15), a mixture containing 1-decene together with the 9-DA ethyl ester (Fig. 16) and the pure fraction with 9-DA ethyl ester (purity > 95%) (Fig. 17).

## Example 12. Distillation of the post-reaction mixture after the MOR ethenolysis reaction.

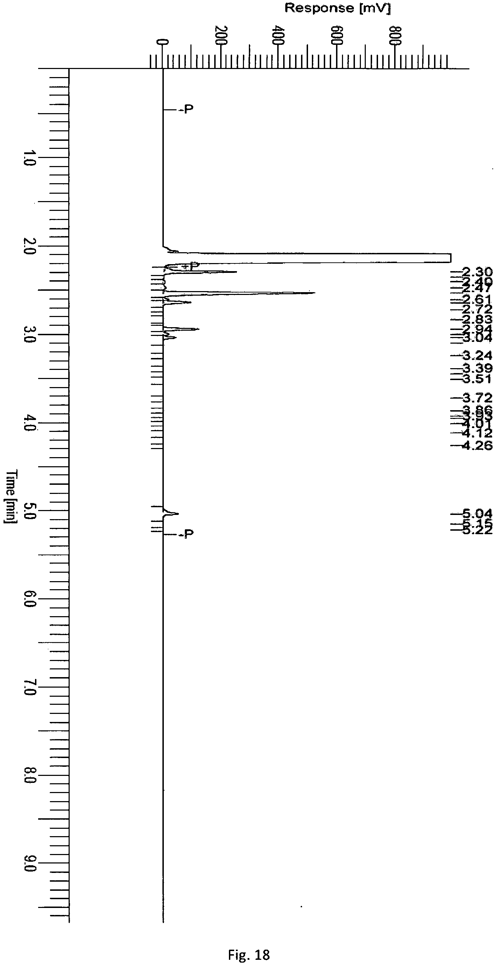

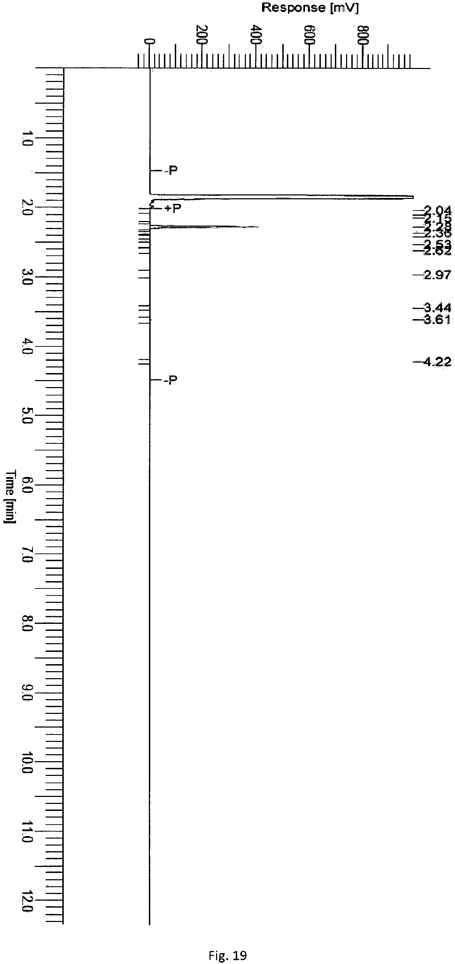

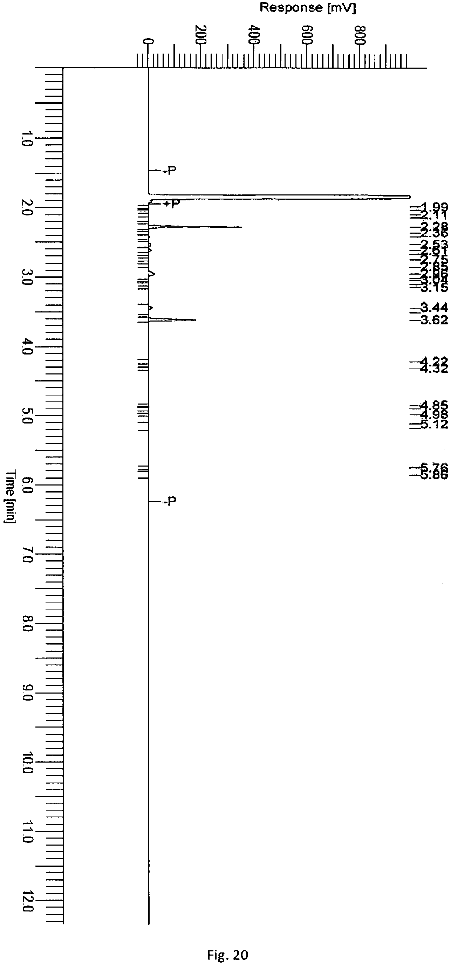

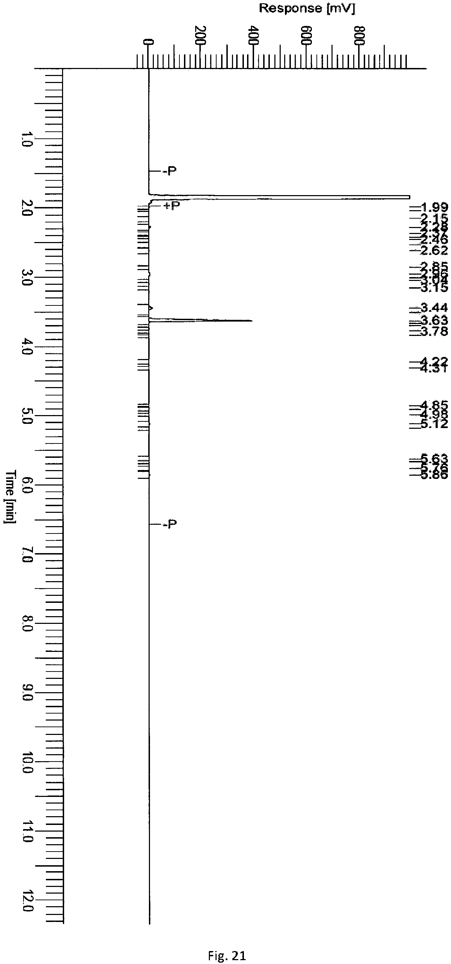

Distillation was carried out in a 1 $dm^3$ flask with 600 g of post-reaction mixture on a 44.5 cm distillation column. The entire distillation was carried out at a pressure of 1-2 mbar. All fractions were analyzed by GC using the following method: initial temperature 145 °C, time 0.1 min, heating 9 °C/min to 180 °C, time 0.1 min, heating 20 °C/min to 305 °C, time 2 minutes. Following fractions were collected: mixed fraction of mainly 1-heptene (Fig. 18), pure 1-decene (purity 98%) (Fig. 19), the mixture containing 1-decene with the 9-decenoic acid methyl ester (Fig. 20) and the pure fraction with 9-decenoic acid methyl ester (purity 92%) (Fig. 21).

## Example 13. Saponification of 9-decenoic acid alkyl esters.

50 $cm^3$ of water was poured into a round bottom flask, followed by the addition of 8 grams (40 mmol) of 9-DA ethyl ester. 2M KOH solution (50 $cm^3$) was carefully added, the reaction mixture was heated to 120 °C, and the reaction was carried out 3 hours until all substrate disappeared (observed by thin layer chromatography - TLC). The reaction mixture was cooled to room temperature. The whole mixture was acidified with 2M hydrochloric acid against the indicator paper until slightly acidic (pH about 6). The acid was extracted with diethyl ether (3x30 $cm^3$) and then dried over sodium sulfate. 6.5 grams of a colorless oily liquid was obtained (95% yield). The structure was confirmed by $^1$H and $^{13}$C NMR, and the obtained results were verified with literature data.

## Claims

1. A method for the preparation of a mixture of ethyl 9-decenoate and 1-decene by the ethenolysis reaction from a raw material of technical purity and containing fatty acid alkyl esters and ethyl oleate content of at least 70%, comprising the following steps:

    a) preparation of the substrate - alkyl ester of oleic acid,
    b) preparation of the catalyst solution,
    c) contacting the catalyst solution with the substrate in the reactor,
    d) filling the reactor with ethylene gas,
    e) mixing the reaction mixture,
    f) pouring down the reaction mixture from the reactor,
    g) fractional distillation of the reaction mixture,
    h) hydrolysis of 9-decenoic acid alkyl esters,

    **characterized in that** in step a) the substrate is contacted with magnesol, and in step b) the catalyst solution is prepared by dissolving in toluene a ruthenium complex containing at least one carbene ligand with a five-membered heterocyclic ring containing two tertiary nitrogen atoms in which one of the nitrogen atoms is substituted with a thiophenomethylene substituent and the other nitrogen atom is substituted with an aromatic substituent containing at least one branched alkyl substituent containing at least three carbon atoms in the alkyl chain and the reaction in step e) is carried out for 2 to 8 hours and at a temperature of 50 °C to 80 °C under ethylene pressure 10 bar to 20 bar and in step f) the reaction mixture is contacted with the scavenger solution.

2. The method according to Claim 1, **characterized in that** the ruthenium complex is selected from the group consisting of: bis[1-(2,6-diisopropylphenyl)-3-(thiophen-2-ylmethyl)imidazolidin-2-yl](3-phenyl-1H-inden-1-ylidene) ruthenium (II) chloride or [1-(2,6-diisopropylphenyl)-3-(thiophen-2-ylmethyl)imidazolidin-2-yl](2-isopropoxybenzylidene) ruthenium (II) chloride.

3. The method according to Claim 1 or 2, **characterized in that** the branched alkyl substituent is an isopropyl substituent.

4. The method according to Claim 1, 2 or 3, **characterized in that** in step b) a homogeneous catalyst solution is prepared in a solvent selected from the group comprising toluene or dichloromethane.

5. The method according to Claim 1, 2, 3 or 4, **characterized in that** the reaction is carried out at a temperature of up to 50 °C in the presence of a monocarbene catalyst.

6. The method according to Claims 1, 2, 3, 4 or 5, **characterized in that** the reaction is carried out at a temperature of 80 °C in the presence of a biscarbene catalyst.

7. The method according to Claims 1, 2, 3, 4, 5 or 6, **characterized in that** the reaction is carried out for up to 2 hours in the presence of a monocarbene catalyst.

8. The method according to Claims. 1, 2, 3, 4, 5, 6 or 7, **characterized in that** the reaction is carried out for up to 8 hours in the presence of a biscarbene catalyst.

9. The method according to Claims 1, 2, 3, 4, 5, 6, 7 or 8, **characterized in that** the reaction is carried out at ethylene pressure of 20 bar.

10. The method according to Claims 1, 2, 3, 4, 5, 6, 7, 8 or 9, **characterized in that** the scavenger is 1,4-bis(3-isocyanopropyl)piperazine.

Fig. 1

Fig. 2

**Ru-1** **Ru-2** **Ru-3** **Ru-4**

Ru-5 (R = H, X =Cl)
Ru-6 (R = H, X = I)
Ru-7 (R = NO$_2$, X = Cl)
Ru-8 (R = NO$_2$, X = I)
Ru-9 (R = NHCOCF$_3$, X = Cl)
Ru-10 (R = NHCOCF$_3$, X = I)
Ru-11 (R = NHCO$_2$$^i$Bu, X = Cl)
Ru-12 ( SO$_2$NMe$_2$, X = Cl)

Ru-13 (R = H, X = Cl)
Ru-14 (R = NO$_2$, X = I)
Ru-15 (R = OMe, X = Cl)
Ru-16 (R = NHCOCF$_3$, X= Cl)
Ru-17 (R = NHCOCF$_3$, X = I)

Ru-18 (X = Cl)
Ru-19 (X = I)

**Ru-20**

Ru-21 (R = Cyclopentyl)
Ru-22 (R = Cyclohexyl)

Ru-23 (R = H)
Ru-24 (R = CH$_3$)
Ru-25 (R = OMe)

**Ru-26** **Ru-27** **Ru-28** **Ru-29** **Ru-30** **Ru-31**

**Ru-32** **Ru-33** **Ru-34** **Ru-35** **Ru-36** **Ru-37**

Ru-38 (R = OCOCF$_3$) **Ru-39** Ru-40 (R = OCOC$_6$F$_5$)

Ru-41 (R = H)
Ru-42 (R = NMe$_2$)

Ru-43 (R = H)
Ru-44 (R = NMe$_2$)

Ru-45 (R = H)
Ru-46 (R = Me)
Ru-47 (R = OMe)
Ru-48 (R = NO$_2$)

Ru-49  Ru-50  Ru-51  Ru-52  Ru-53  Ru-54

Ru-55 (R = H)
Ru-56 (R = OMe)
Ru-57 (R = NMe₂)

Ru-58 (R = H)
Ru-59 (R = OMe)
Ru-60 (R = NMe₂)

Ru-61

Ru-62

Ru-63 (X = Cl)
Ru-64 (X = I)

Ru-65

Fig.3

Fig. 4

Fig. 5

Fig. 6

Fig. 7

Fig. 8

Fig. 9

Fig. 10

Fig. 11

Fig. 12

Fig. 13

## calibration EO

$y = 28462x - 38562$
$R^2 = 0,9976$

Fig. 14

Fig. 15

Fig. 16

Fig. 17

Fig. 18

Fig. 19

Fig. 20

Fig. 21

Europäisches
Patentamt

European
Patent Office

Office européen
des brevets

## EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2016/092424 A1 (UNIV WARSZAWSKI [PL]) 16 June 2016 (2016-06-16) * page 8; compounds 1ab,1bb * * page 35 - page 36; example XVIII * | 1-10 | INV. C07C67/333 C07C69/533 C07C6/04 C07C11/02 |
| A | WO 2008/046106 A2 (MATERIA INC [US]; SCHRODI YANN [US] ET AL.) 17 April 2008 (2008-04-17) * example 12 * | 1 | |
| A | WO 2017/055945 A1 (APEIRON SYNTHESIS SA [PL]) 6 April 2017 (2017-04-06) * example XXIII * | 1 | |
| A | US 2014/275580 A1 (LITTICH RYAN A [US] ET AL) 18 September 2014 (2014-09-18) * example 3 * | 1 | |
| A | WO 2008/010961 A2 (MATERIA INC [US]; SCHRODI YANN [US]) 24 January 2008 (2008-01-24) * paragraphs [0079], [0088], [0089] * * examples * * claim 21 * | 1,9 | TECHNICAL FIELDS SEARCHED (IPC) C07C |

-/--

The present search report has been drawn up for all claims

| Place of search | Date of completion of the search | Examiner |
|---|---|---|
| The Hague | 15 April 2019 | Kardinal, Siegmar |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

Europäisches
Patentamt

European
Patent Office

Office européen
des brevets

# EUROPEAN SEARCH REPORT

Application Number

EP 18 46 1623

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MICHAL SMOLEN ET AL: "Ruthenium Complexes Bearing Thiophene-Based Unsymmetrical N-Heterocyclic Carbene Ligands as Selective Catalysts for Olefin Metathesis in Toluene and Environmentally Friendly 2-Methyltetrahydrofuran", CHEMISTRY - A EUROPEAN JOURNAL, vol. 24, no. 57, 19 September 2018 (2018-09-19), pages 15372-15379, XP055580692, DE ISSN: 0947-6539, DOI: 10.1002/chem.201803460 * the whole document * | 1-10 | |
| L | PRZEMYSLAW WYREBEK ET AL: "Looking for the Noncyclic(amino)(alkyl)carbene Ruthenium Catalyst for Ethenolysis of Ethyl Oleate: Selectivity Is on Target", ACS OMEGA, vol. 3, no. 12, 27 December 2018 (2018-12-27), pages 18481-18488, XP055581001, ISSN: 2470-1343, DOI: 10.1021/acsomega.8b03119 * the whole document * | 1-10 | TECHNICAL FIELDS SEARCHED (IPC) |

The present search report has been drawn up for all claims

| Place of search | Date of completion of the search | Examiner |
|---|---|---|
| The Hague | 15 April 2019 | Kardinal, Siegmar |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 46 1623

This annex lists the patent family members relating to the patent documents cited in the above-mentioned European search report.
The members are as contained in the European Patent Office EDP file on
The European Patent Office is in no way liable for these particulars which are merely given for the purpose of information.

15-04-2019

| Patent document cited in search report | | Publication date | Patent family member(s) | | Publication date |
|---|---|---|---|---|---|
| WO 2016092424 | A1 | 16-06-2016 | EP | 3294747 A1 | 21-03-2018 |
| | | | WO | 2016092424 A1 | 16-06-2016 |
| WO 2008046106 | A2 | 17-04-2008 | CN | 102123979 A | 13-07-2011 |
| | | | CN | 106083579 A | 09-11-2016 |
| | | | EP | 2076484 A2 | 08-07-2009 |
| | | | US | 2010145086 A1 | 10-06-2010 |
| | | | US | 2013035532 A1 | 07-02-2013 |
| | | | WO | 2008046106 A2 | 17-04-2008 |
| WO 2017055945 | A1 | 06-04-2017 | AU | 2016330158 A1 | 12-04-2018 |
| | | | CA | 2999412 A1 | 06-04-2017 |
| | | | CN | 108137631 A | 08-06-2018 |
| | | | EP | 3356379 A1 | 08-08-2018 |
| | | | JP | 2018535194 A | 29-11-2018 |
| | | | KR | 20180059822 A | 05-06-2018 |
| | | | US | 2018298046 A1 | 18-10-2018 |
| | | | WO | 2017055945 A1 | 06-04-2017 |
| US 2014275580 | A1 | 18-09-2014 | CN | 105051020 A | 11-11-2015 |
| | | | EP | 2970169 A1 | 20-01-2016 |
| | | | US | 2014275580 A1 | 18-09-2014 |
| | | | US | 2015307469 A1 | 29-10-2015 |
| | | | WO | 2014159226 A1 | 02-10-2014 |
| WO 2008010961 | A2 | 24-01-2008 | US | 2008027194 A1 | 31-01-2008 |
| | | | US | 2012071676 A1 | 22-03-2012 |
| | | | US | 2013289327 A1 | 31-10-2013 |
| | | | US | 2014200382 A1 | 17-07-2014 |
| | | | US | 2016185689 A1 | 30-06-2016 |
| | | | WO | 2008010961 A2 | 24-01-2008 |

EPO FORM P0459

For more details about this annex : see Official Journal of the European Patent Office, No. 12/82

**EP 3 650 436 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007270621 A1
- US 2014336399 A1
- WO 02076920 A1
- WO 2011056881 A2
- EP 3294747 A1